# EUROPEAN PATENT SPECIFICATION

(11) **EP 1 317 186 B1**
(45) Date of publication and mention of the grant of the patent: **12.01.2011**
(21) Application number: 01970375.0
(22) Date of filing: 11.09.2001
(51) Int. Cl.: A23J 3/34

(54) **IMPROVED BIOACTIVE WHEY PROTEIN HYDROLYSATE**
VERBESSERTES BIOAKTIVES MOLKEPROTEINHYDROLYSAT
HYDROLYSAT PROTEIQUE BIOACTIF AMELIORE DE LACTOSERUM

(30) Priority: 11.09.2000 NZ 50686600
(43) Date of publication of application: 11.06.2003
(73) Proprietor: NEW ZEALAND DAIRY BOARD, Wellington 1 (NZ)
(72) Inventor: SCHLOTHAUER, Ralf-Christian, Palmerston North (NZ); SCHOLLUM, Linda, May, Palmerston North (NZ); REID, Julian, Robert, Palmerston North (NZ); HARVEY, Stephanie, Adele, Palmerston North (NZ); CARR, Alistair, Palmerston North (NZ); FANSHAWE, Rachel, Lois, Lyall Bay Wellington (NZ)
(74) Representative: Barton, Matthew Thomas
(86) International application number: PCT/NZ2001/000188
(87) International publication number: WO 2002/019837

(56) References cited:
- EP-B1- 0 269 593
- WO-A-00/42863
- WO-A1-91/13554
- WO-A1-92/21248
- WO-A1-93/04593
- WO-A1-93/24020
- WO-A1-99/65326
- JP-A- 10 033 115
- US-A- 5 952 193
- US-A- 6 060 269
- MUTILANGI W A M ET AL: "FUNCTIONAL PROPERTIES OF HYDROLYSATES FROM PROTEOLYSIS OF HEAT- DENATURED WHEY PROTEIN ISOLATE" JOURNAL OF FOOD SCIENCE, INSTITUTE OF FOOD TECHNOLOGISTS, CHICAGO, IL, US, vol. 61, no. 2, 1 March 1996 (1996-03-01), pages 270-274,303, XP000589497 ISSN: 0022-1147
- PIHLANTO-LEPPALA ANNE ET AL: "Angiotensin I-converting enzyme inhibitory properties of whey protein digests: Concentration and characterization of active peptides" JOURNAL OF DAIRY RESEARCH, vol. 67, no. 1, February 2000 (2000-02), pages 53-64, XP008022966 ISSN: 0022-0299
- CASTRO ET AL.: 'Proteolysis of whey proteins by a bacillus subtilis enzyme preparation' INT. DAIRY J. vol. 6, no. 3, 1996, pages 285 - 294, XP000853722

## Description

### TECHNICAL FIELD

This invention relates to a process for producing improved hydrolysed whey protein products which are substantially free of bitter flavours and which contain bioactive peptides. The products of the process have high digestibility and good organoleptic properties. The products have a bland taste and are free of soapy or brothy flavours. The improved hydrolysed whey protein products are useful sources of bioactive peptides for incorporation into functional foods.

### BACKGROUND ART

A number of food ingredients and foodstuffs have been produced from the hydrolysis of a protein source such as the milk proteins, casein and whey proteins.

Hydrolysed protein foodstuffs may have advantages over non-hydrolysed protein foodstuffs in a number of areas of health care. For example, it is known that enzymatically hydrolysed proteins are less allergenic. They are also more rapidly digested and absorbed than whole proteins. Foodstuffs containing hydrolysed proteins are also useful in the alimentation of hospital patients with digestive diseases for example.

Hydrolysis of whey proteins and caseins is known to release bioactive peptides that can exhibit a number of physiological effects (Maubois et al, 1991; EP 4745506). A number of publications describe such bioactive peptides, for example, ACE inhibiting peptides which have antihypertensive properties have been released through an enzymatic treatment of β-lactoglobulin and whey protein concentrates (Mullally et al, 1997). ACE inhibitory peptides are also found in sour milk and in hydrolysates of αₛ and β casein (JP 4282400; Nakamura et al 1994, Yamamoto 1997).

EP 4745506 discloses the hydrolysis of the milk protein lactoferrin in whey to release lactoferricin which acts as an antimicrobial agent useful for treating diarrhoea, athlete's foot, eye infections, mastitis etc in humans and animals.

However, the hydrolysis of most food proteins, especially the hydrolysis of whey and casein containing products, is known to generate bitterness. This causes palatability problems particularly when attempting to formulate orally ingestible products incorporating milk protein hydrolysates as a source of bioactive peptides.

In the field of protein hydrolysis one or both of two approaches are commonly used for controlling or removing bitterness in protein hydrolysates to increase palatability of the products.

The extensive hydrolysis of the protein substrate is known to reduce bitterness in milk protein hydrolysates (EP 065663; EP 0117047; US 3970520). Less bitter products are produced relatively easily and cheaply in this way. However, extensive hydrolysis reduces the chain lengths of all peptides, including the bioactive peptides of interest. Extensive hydrolysis of the protein substrate destroys the functional and biological activity of the peptide of interest. In addition soapy and brothy off-flavours often develop, with the consequence that the palatability of the final product remains poor compared to the original bland tasting protein substrate. A final disadvantage is that for some hydrolysates the bitterness is only partially removed (Roy 1992 and 1997).

A second common method for the control of bitterness in protein hydrolysates is to use debittering enzymes, in particular those sourced from *Aspergillus oryzae.*

"Bitterness" generation in protein hydrolysis is thought to be due to the presence of large hydrophobic 'bitter' peptides. Debittering enzymes selectively hydrolyse bitter peptides present in the protein hydrolysates. A worker skilled in the art can - by the judicious selection of debittering enzymes and the conditions of treatment - effectively debitter milk protein hydrolysates leaving intact the particular bioactive peptides of interest. However, use of debittering enzymes makes the process more expensive, and preservation of some of the bioactive peptide is not easily or successfully achieved. A further disadvantage is that debittering enzymes treatments have a tendency to release free amino acids into the final product and, as a consequence, the hydrolysates develop unpleasant brothy or soapy flavours (Roy 1992 and 1997).

The various methods of debittering the protein hydrolysates result in additional process steps and add costs to the manufacture of the final product. In addition the final product also becomes overbalanced in its supply of free amino acids.

It would be most advantageous if a process for hydrolysing protein could be developed which releases bioactive peptides of interest and which limits the formation of bitter peptides and free amino acids, thereby allowing the original bland taste of the milk protein substrates to be retained.

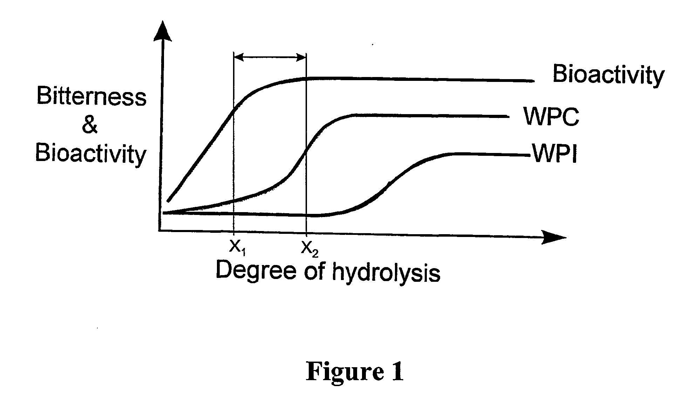

Some bioactive peptides - in particular the antihypertensive peptides - are relatively stable during protein hydrolysis and are released very early during the hydrolysis of the milk protein substrate as shown in Figure 1.

The bitter flavours of milk protein hydrolysates can be improved by adding sugars or by hydrolysing natural sugars, such as lactose, already present in the milk protein substrate (Bernal and Jelen, 1989). For example sour wheys and cheese wheys are made more palatable when they have been sweetened by β-galactosidease and lactase hydrolysis of lactose (FR 2309154; US 4358464; JP 8056568).

In order to achieve a high flavour acceptability for a hydrolysed protein product which contains bioactive peptides, precise control of hydrolysis is required to prevent bitterness occurring.

A common method of termination of hydrolysis is by deactivation of the enzymes, usually by thermal deactivation at high temperatures, typically> 90-100°C for an extended period of time. However, this method cannot be used to stop the hydrolysis of whey proteins as any intact unhydrolysed whey proteins remaining in the mixture would denature and precipitate making the final product less soluble and less acceptable for the use as a food ingredient.

Such a problem was overcome in WO 99/65326 which discloses a process of mild hydrolysis of sweet whey or sweet whey protein concentrate (WPC) to produce hydrolysates containing bioactive peptides having one or more of the following properties:
- antihypertensive ACE-I activity
- bifidus growth promoting activity
- non-gluey, non-bitter flavour
- pleasant to slightly sweet taste
- good organoleptic properties.

The present invention uses a different whey protein-containing substrate to that used in WO 99/65326 although a similar hydrolysing process is used. Surprisingly, the use of this different substrate results in a hydrolysate which shows dramatic improvements in the above mentioned properties of the whey hydrolysates, particularly in the antihypertensive ACE-1 activity, flavour and functionality of the product.

It is broadly to the process of hydrolysing a different whey protein containing substrate and the novel hydrolysate produced by this process that the present invention is directed.

### SUMMARY OF THE INVENTION

The present invention provides a process for preparing an improved whey protein hydrolysate containing bioactive peptides comprising hydrolysing a whey protein isolate (WPI) with one or more enzymes **characterised in that**:
i) the enzyme is a heat labile protease;
ii) the hydrolysis is conducted at a temperature of between about 30°C and 65°C at a pH of about 3.5 to about 9.0 when said enzyme is a neutral protease, at a pH of about 2.5 to about 6.0 where said enzyme is an acid protease;
iii) the hydrolysis is terminated when a degree of hydrolysis of no greater than about 10% has been reached; and
iv) the hydrolysis is terminated by deactivating said one or more enzymes under conditions to avoid substantial denaturation of peptides or residual proteins in said hydrolysate, wherein said deactivation conditions are selected from the group consisting of; heat deactivation comprising heating said hydrolysate for up to ten seconds to a temperature up to about 100°C;
or when said hydrolysis is conducted at a temperature of below about 65°C, said heat deactivating step is conducted at about 65°C to about 70°C for from about 10 seconds to about 15 minutes; or when said hydrolysis is conducted at a temperature below about 60°C, said heat deactivation is conducted at about 60°C to about 65°C for from about 10 seconds up to about 30 minutes;
or by altering the pH of said whey-protein containing substrate to a pH at which said protease is not active; or by subjecting said hydrolysate to ultrafiltration with an ultrafiltration membrane having a nominal molecular weight cutoff in the range of about 10-500 kDa, wherein the product of the process is highly soluble.

By WPI is meant a whey protein isolate produced by any method known in the art. Preferably the WPI is produced by a method of ion exchange from a whey protein concentrate, such as a cheese, acid, or lactic whey protein concentrate, as would be appreciated by a person skilled in the art.

By heat labile is meant that the enzyme is susceptible to irreversible deactivation at relatively moderate temperatures as would be appreciated by a person skilled in the art.

Preferably the enzyme is selected from the group consisting of Protease P6, Protease A, Protease M, Peptidase, Neutrase, Validase, AFP 2000, and any other heat labile protease.

The enzyme hydrolysis step may be carried out under conditions which are suitable for the particular enzyme used as would be understood by a person skilled in the art.

Preferably, said ultrafiltration membrane has a nominal molecular weight cut off in the range of about 10-200 kDa.

Advantageously, said enzyme is immobilised on an inert support during said hydrolysis step ii).

Conveniently, the inert support is a methacrylate-based functional polymer, carrageenan particles, chitosan particles or any other suitable inert support material.

Preferably, the degree of hydrolysis is from about 3% to about 10%.

Advantageously, the degree of hydroysis is from about 3% to about 5%.

Conveniently, the whey protein hydrolysate so produced comprises one or more bioactive peptides selected from the group consisting of SAP (SEQ ID NO: 1), VSLPEW (SEQ ID NO: 5), INYWL (SEQ ID NO: 6), LKPTPEGDLEIL (SEQ ID NO: 7) and LKGYGGVSLPEW (SEQ ID NO: 8).

Preferably, the whey protein hydrolysate so prepared comprises at least one bioactive peptide selected from the group consisting of LIVTQ (SEQ ID NO: 4), MKG (SEQ ID NO: 2) and ALPMH (SEQ ID NO: 3), in combination with at least one bioactive peptide selected from the group comprising SAP (SEQ ID NO: 1), VSLPEW (SEQ ID NO: 5), INYWL (SEQ ID NO: 6), LKPTPEGDLEIL (SEQ ID NO: 7) and LKGYGGVSLPEW (SEQ ID NO: 8).

The present invention also provides a pharmaceutical composition comprising at least one bioactive peptide selected from the group consisting of SAP (SEQ ID NO: 1), VSLPEW (SEQ ID NO: 5), INYWL (SEQ ID NO: 6), LKPTPEGDLEIL (SEQ ID NO: 7) and LKGYGGVSLPEW (SEQ ID NO: 8) in combination with the bioactive peptide MKG (SEQ ID NO: 2) together with a pharmaceutically acceptable carrier.

Furthermore, the invention provides a non-bitter, highly soluble WPI hydrolysate product containing bioactive peptides, prepared by the process of claim 8 or 9.

Advantageously, the degree of hydrolysis of the WPI is about 3% to about 5%.

Conveniently, the main particle size of whey proteins in the product is less than about 30 microns.

Preferably, the main particle size is less than about 3 microns.

Advantageously, a product is clear or white in solution.

Conveniently, one or more of said bioactive peptides is selected from the group consisting of SAP (SEQ ID NO: 1), VSLPEW (SEQ ID NO: 5), INYWL (SEQ ID NO: 6), LKPTPEGDLEIL (SEQ ID NO: 7) and LKGYGGVSLPEW (SEQ ID NO: 8).

Preferably, at least one bioactive peptide selected from the group consisting of LIVTQ (SEQ ID NO: 4), MKG (SEQ ID NO: 2) and ALPMH (SEQ ID NO: 3), in combination with at least one bioactive peptide selected from the group comprising SAP (SEQ ID NO: 1), VSLPEW (SEQ ID NO: 5), INYWL (SEQ ID NO: 6), LKPTPEGDLEIL (SEQ ID NO: 7) and LKGYGGVSLPEW (SEQ ID NO: 8).

The invention also provides a food product containing a WPI hydrolysate product according to the invention.

There is also provided the use of a product prepared by the invention in the manufacture of a medicament for treating or preventing hypertension in a patient in need thereof.

Furthermore, the invention provides a pharmaceutical composition comprising the product the invention together with a pharmaceutically acceptable carrier.

In addition, the invention provides a hydrolysate comprising one or more peptides selected from SAP (SEQ ID NO: 1), VSLPEW (SEQ ID NO: 5), INYWL (SEQ ID NO: 6), LKPTPEGDLEIL (SEQ ID NO: 7) and LKGYGGVSLPEW (SEQ ID NO: 8).

Furthermore, the invention provides a hydrolysate comprising a mixture of at least one first peptide and at least one second peptide, wherein the first peptide is selected from the group consisting of LIVTQ (SEQ ID NO: 4), MKG (SEQ ID NO: 2) and ALPMH (SEQ ID NO: 3), and wherein the second peptide is selected from the

group comprising SAP (SEQ ID NO: 1), VSLPEW (SEQ ID NO: 5), INYWL (SEQ ID NO: 6), LKPTPEGDLEIL (SEQ ID NO: 7) and LKGYGGVSLPEW (SEQ ID NO: 8).

The invention also provides a pharmaceutical composition comprising the hydrolysate according to the invention together with a pharmaceutically acceptable carrier.

In addition the invention provides a use of the hydrolysate of the invention for the manufacturer of a medicament for treating or preventing hypertension in a patient in need of such treatment.

The invention further comprises the step of combining a pharmaceutically acceptable carrier to produce a pharmaceutical composition.

The hydrolysed WPI product of the invention has one or more of the following features:
- antihypertensive ACE-I activity
- probiotic growth promoting activity
- non-gluey, non-bitter flavour
- pleasant to slightly sweet taste
- good organoleptic properties
- high solubility
- very good foaming properties
- very good gelling properties
- improved heat stability

The application of the mild hydrolysis technology to the substrate of whey protein isolates (WPI) revealed some dramatic improvements of the final product in comparison to sweet whey protein concentrate (WPC) as a whey-protein containing substrate as disclosed in WO 99/65326 as follows:
- Solubility
   Although the WPI still denatures slightly on the heat conditions to stop the protease reaction, it does not produce any insoluble material. The solubility remains around 96% which is greater than the corresponding WPC hydrolysate.
- Heat stability
   The hydrolysed WPI was significantly more heat stable than the hydrolysed WPC. After 120°C for 10 min @ 5% TS the solubility remains 95%.
- Appearance
   Proper selection of reaction conditions can determine whether the final hydrolysate will look white or clear in solution. Example 1, below, produces an opaque product, whilst Examples 2 and 3 (below) result in clear product in solution at neutral pH. Hydrolysates of WPC were all substantially white in appearance.
- Foaming ability and stability
   The hydrolysed WPI shows about double the foaming ability and about four times the foam stability of non-hydrolysed WPI. This makes the product very suitable as an ingredient for yoghurt and dessert applications. The hydrolysed WPI also showed improved foaming ability and stability compared to a WPC hydrolysate.
- Gel strength
   The mildly hydrolysed WPI shows a markedly increased gel strength compared to non-hydrolysed WPI. This makes the product very suitable as an ingredient for yoghurt and dessert applications. The hydrolysed WPI also showed improved gel strength compared to a WPC hydrolysate.
- Flavour
   The hydrolysed WPI shows significantly less bitterness compared to mildly hydrolysed WPC products. The bitterness shows no tendency to increase over time of hydrolysis, making the control of the process much easier in comparison to WPC hydrolysis.
- ACE-I activity in vitro
   The hydrolysed WPI shows about double the ACE-I activity in vitro of mildly hydrolysed WPC, given comparable reaction conditions and enzyme addition.
- Acceleration of probiotic fermentation
   The hydrolysed WPI shows an acceleration in the rate of probiotic yoghurt fermentation time of about 40% at an addition rate of 1.5%.

The invention consists in the foregoing and also envisages constructions of which the following gives examples.

### BRIEF DESCRIPTION OF THE DRAWINGS

The present invention will now be described with reference to the accompanying drawings in which:
- Figure 1: is a plot of bitterness and bioactivity on the ordinant against the degree of hydrolysis on the abscissa. The 'opportunity window' of obtaining a product according to the present invention containing bioactive peptides and having acceptable flavours before the hydrolysis reaction produces bitter peptides is between the lines x₁ and x₂.
- Figure 2: shows the Acute Short Term Effect of Systolic Blood Pressure of dosing mature spontaneously hypertensive rates (SHRs) with 6.0g WPC hydrolysate/kg body weight.
- Figure 3: shows the Acute Short Term Effect of Systolic Blood Pressure of dosing mature spontaneously hypertensive rates (SHRs) with 3.6g WPI hydrolysate/kg body weight.
- Figure 4: shows the Acute Short Term Effect of Systolic Blood Pressure of dosing mature spontaneous hypertensive rates (SHRs) with 165mg peptide MKG (SEQ ID NO: 2)/kg body weight.

### DETAILED DESCRIPTION OF THE INVENTION

As discussed above, the present invention provides a process for producing a hydrolysed WPI product containing bioactive peptides, whereby the hydrolysis is carried out under a high degree of control to prevent undesirable flavours developing during hydrolysis (e.g. bitter, soapy and brothy). The hydrolysis is terminated within the "opportunity window", i.e. before the emergence of substantial bitterness - as shown in Figure 1 - to provide hydrolysates having good organoleptic properties and maximum bioactive peptides. In Figure 1 the degree of hydrolysis is represented qualitatively on the x axis. The window of opportunity is between the points x₁ and x₂ which will vary depending on the enzyme which is used. The optimum conditions sought are a maximum bioactivity with an acceptable level of bitterness.

In particularly preferred embodiments of the process of the invention, the enzyme which hydrolyses the WPI is heat labile and is selected from the group consisting of Protease P6, Protease A, Protease M, Peptidase, Neutrase, Validase and AFP 2000 (all as herein defined) and the hydrolysis of the WPI is terminated by heat treatment for a short time at a high temperature (about 85-100°C for about 1-10 seconds). The applicants have surprisingly found that the above enzymes (1) are able to produce a whey protein hydrolysate containing a good level of bioactive peptides, and (2) can be inactivated by a short time, high temperature treatment which causes only partial denaturation of the whey proteins in the hydrolysate, and surprisingly improves the organoleptic properties of the whey proteins, in terms of providing a product which is substantially white or clear in appearance.

The present invention is now exemplified by the following examples using ALACEN™ 895 or ALACEN™ 894 (whey protein isolates, commercially available from NZDB), the product specifications for which are attached in Appendix I:

### Example 1

### Pilot plant production of WPI mild hydrolysate

Whey protein isolate produced by cation ion exchange technology (ALACEN™ 895) with a protein content ≥ 90% w/w was reconstituted at 20% total solids in water (50°C) Reconstituted ALACEN^{™} 895 was transferred to a 150L tank at 50°C. Water (50°C) was added to the tank to make final total solids of 4%. The solution was stirred and Neutrase (E:S 0.9%) was added.

Two hours after enzyme addition the first hydrolysate was pumped through the UHT plant. Enzyme inactivation was achieved using direct steam injection to heat the hydrolysate to 88°C and the hydrolysate was held it at this temperature for 1.5 seconds. The hydrolysate was flash cooled and passed through shell and tube heat exchanges to cool to ambient temperature.

The hydrolysate was subsequently evaporated and dried.

A hydrolysate made following the process of Example 1 had the following features:
Solubility: 95%
Heat stability: 120°C for 10 min @ 5% TS solubility 95%
ACE-I in vitro activity: 289 mg/L IC50
Foaming: Markedly increased over non-hydrolysed WPI
Flavour: Markedly improved over WPC based mild hydrolysates
Appearance: opaque white, particle size ∼ 0.1 µm

The solubility, heat stability, foaming, and appearance of the WPI hydrolysate were measured by standard methods familiar to those skilled in the art. The solubility of 5% total solids (TS) solution was determined by centrifugation at 3000 g for 10mins (at room temperature). The heat stability of 5% TS solutions was determined by heating to 120°C for 10mins, quick cooling, then centrifugation at 700 g for 10mins. The TS contained in the supernatant and the original solution was determined. The solubility was defined as the TS(supernatant)/TS(original solution). Foaming of 10% TS solutions at pH 7.0 was determined by whisking with a Hobart mixer (Model N-50G, Hobart Corporation) for 15 mins. The percentage overrun was used to compare the sample as would be understood by a person skill in the art. The appearance of 5% TS was determined by visual observation and the particle size was measured using a Malvern MasterSizer (Model MSE00SM, Malvern Instruments Ltd).

ACE-1 activity (*in vitro*) in the dried product was determined using FAPGG as a substrate (Product 305-10 ex Sigma Chemical Corporation, St Louis, MO, USA) according of the method of D W Cushman & H S Cheung (1971). ACE-1 activities are expressed as the amount of material (mg/L) needed to reduce the activity of the ACE-1 enzyme by 50%.

The flavour was assessed subjectively with reference to the bitterness and astringency of the hydrolysates, in particular, the sensory profile was assessed by a formal sensory panel. 5% w/w samples were tasted at 24°C using multi-dimensional scaling.

Samples were evaluated and scored on a 150mm anchored line (absent (0) to intense (150)).

### Example 2

The hydrolysis reaction was repeated as outlined above for Example 1 (10% total solids, E:S 0.9%, 150L). After inactivation the hydrolysate was immediately evaporated and dried

A hydrolysate made by this method had the following features which were measured as disclosed above for Example 1:
Solubility: 97%
Heat stability: 120°C for 10 min @ 5% TS solubility 96%
ACE-1 in vitro activity: 503 mg/L IC50
Foaming: Markedly increased over non-hydrolysed WPI
Gelling: Markedly increased over non-hydrolysed WPI
Flavour: Markedly improved over WPC based mild hydrolysates
Appearance: clear yellowish

### Example 3

The hydrolysis reaction of Example 1 was repeated as outlined above (4% total solids, E:S 0.9%, 150 L). Four hours after enzyme addition the first hydrolysate was pumped through the UHT plant using the same conditions as in Example 1.

A hydrolysate made by this method had the following features which were measured as disclosed above for Example 1:
ACE-I in vitro activity: 230 mg/L IC50
Foaming: Markedly increased over non-hydrolysed WPI
Gelling: Markedly increased over non-hydrolysed WPI
Flavour: Markedly improved over WPC based mild hydrolysates
Appearance: clear yellowish

### Example 4

A 2% solution of ALACEN™ 894 (micro-filtered WPI) was altered to pH 3.0 before undergoing ultrafiltration at 10°C with a 3,000 Dalton nominal molecular weight cutoff membrane (CDUF001LB, Millipore Corporation, Bedford). The pH of the retentate was altered to 7.0 and diluted to 2% total solids before ultrafiltration at 10°C with the same membrane used previously. The total solids of the retentate were adjusted to 5.0% before being hydrolysed with E:S 0.9% w/w Neutrase (Novo Nordisk, Denmark) at 50°C. After 4h the sample was inactivated at 88°C for 3 seconds and subsequently freeze-dried. The ACE-1 activity was determined to be 227 mg/L.

### Example 5

Mildly hydrolysed WPI from Example 3 was shown to promote the growth of a probiotic microorganism when added to half fat milk at an addition rate of 1.5%. The milk was heat treated @ 90°C for 10 minutes. After cooling to 37°C the milk was inoculated with 0.1% *S. thermophilus* and 2% *L. rhamnosus* HN001(DR20™). The fermentation time of the control was 20h to reach the required pH of 4.4. In contrast, the WPI hydrolysate addition reduced the fermentation time to 13h.

### Example 6

### Identification of ACE-inhibitor peptides in WPI hydrolysates

The objective of this example was to isolate and identify ACE-inhibitor peptides present in the WPI hydrolysates

### Methodology

- The starting material for ACE-I peptide isolation was a UF permeate (10 kDa) obtained from the original hydrolysate of Example 1;
- Peptides present in the hydrolysate were separated using reverse-phase HPLC;
- Purified peptides were assayed individually for ACE-inhibitor activity as described in Example 1;
- The amino acid sequence of each active peptide was identified by a combination of mass spectrometry and N-terminal sequence analysis;
- The origin of the active peptides was determined by comparing their sequences with the known sequences of the milk proteins.

The peptides, their origins, activities and known similarities are set out in Table 1 below:

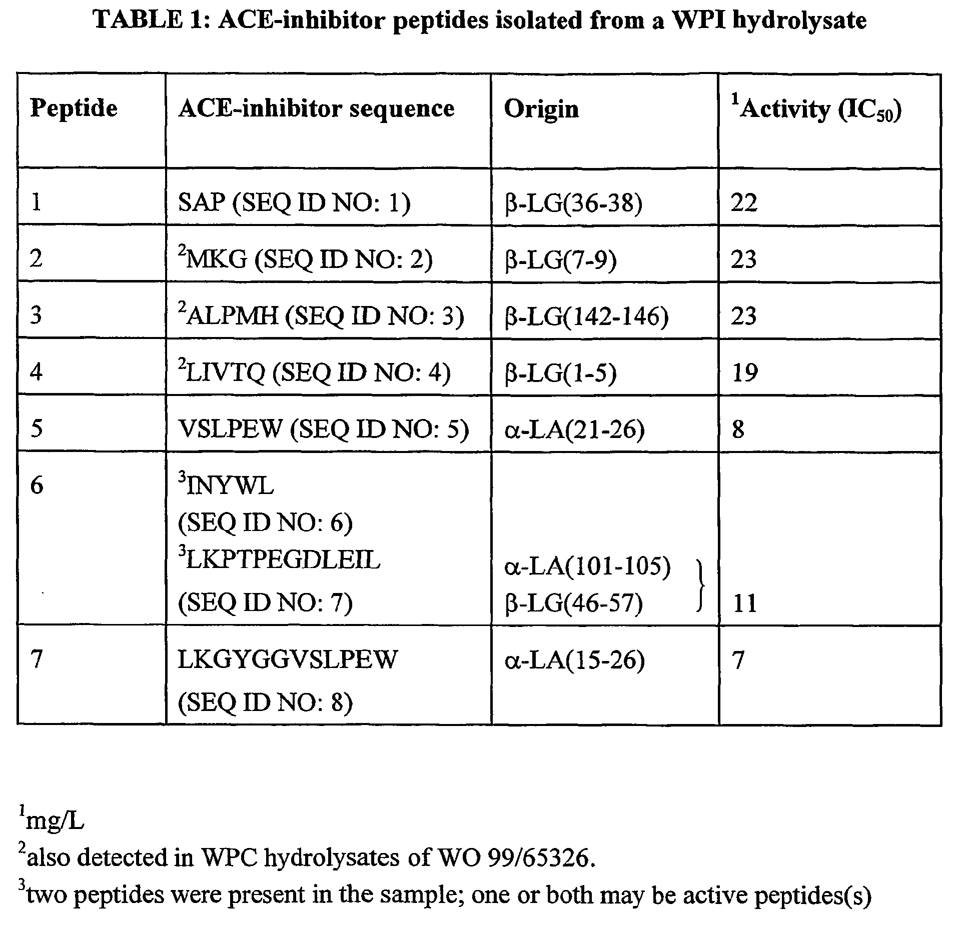

- Three of the active peptides from the WPI hydrolysate were previously identified in the WPC hydrolysate i.e. β-LG(7-9), β-LG(142-146) and β-LG(1-5).

### Example 7

### Sensory Comparison of mildly hydrolysed WPI compared to mildly hydrolysed WPC and unhydrolysed WPI

WPI was hydrolysed as set out above in Example 1.

WPC was hydrolysed as set out in Example 1 of WO 99/65326. (ALACEN^{™} 392, 10%, hydrolysed and inactivated after 2 hours).

ALATAL^{™} 819 is a commercial product of the New Zealand Dairy Board; the summary of the product specification of which is attached in Appendix I.

A taste panel scored the bitterness and astringency of the products scored on a 150mm anchored line (absent (0) to intense (150)).

Degree of Hydrolysis was determined using the Modified O-phthaldialdehyde (MOPA) method as described by Frister *et al.* (1988).

**Table 2 Mean score (/150) for WPI and hydrolysate samples evaluated by a formal sensory panel, degree of hydrolysis, and ACE-I activity of standard WPI and hydrolysed whey protein.**

| **Product tested** | **Sensory Evaluation** | | **Degree of Hydrolysis (%)** | **ACE-I activity (mg/L)** |
|---|---|---|---|---|
| | **Bitter** | **Astringent** | | |
| ALACEN^{™} 895 (WPI) | 36.2 | 24 | 0 | ∼2000 |
| Mildly hydrolysed WPC | 78 | 67.1 | 4.5 | 440 |
| Mildly hydrolysed WPI | 55.1 | 41.6 | 4.3 | 230 |
| ALATAL^{™} 819 | 93.1 | 53.3 | 10 | - |

Both mildly hydrolysed WPI and mildly hydrolysed WPC were more bitter and astringent than the non-hydrolysed WPI product as expected. However, these products were significantly less bitter and astringent than a more vigorously hydrolysed ALATAL^{™} 819 (a product of the New Zealand Dairy Board, NZ).

Mildly hydrolysed WPI products gave more acceptable flavoured products and had significantly higher ACE-I activity, compared to the mildly hydrolysed WPC (described in WO 99/65326). The results also showed that the mildly hydrolysed WPI was significantly less bitter and astringent than the equivalent WPC product, although the degree of hydrolysis for both was similar. As well as having a more acceptable flavour profile, the ACE-I activity of the mildly hydrolysed WPI was almost twice that of the mildly hydrolysed WPC.

Although further hydrolysis of WPC may result in an increased ACE-I activity, a significant decrease in the flavour profile compared to the mildly hydrolysed WPI is observed, compromising the flavour profile due to increased bitterness and astringency.

### Example 8

### Blood Pressure Measurement

Blood pressure was measured using a tail cuff monitor and a purpose designed apparatus for measuring the blood pressure of small animals [IITC Inc, 239 Victory Blvd., Woodland Hills, CA 91367, USA]. Each time point was the average of 3-5 readings taken within approximately 5-10 minutes.

### The Animal Strain

Spontaneously hypertensive rats (SHRs) were sourced from the Animal Resource Centre, Western Australia.

### Sample Dosage

The test substances were given to each rat on a per kg body weight basis unless otherwise stated. The individual dose for each animal was incorporated into a flavoured jelly, which was readily consumed by the animals.

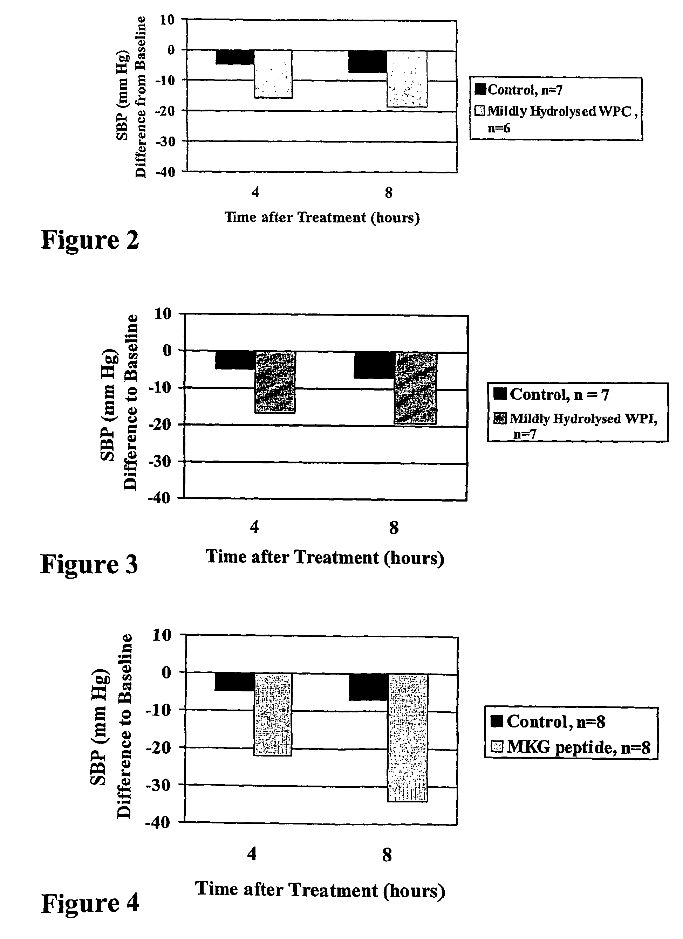

The three samples evaluated for short term blood pressure response were a mildly hydrolysed whey protein concentrate (Figure 2), a mildly hydrolysed whey protein isolate (see Figure 3) and a synthesised peptide from Table 1, namely MKG (SEQ ID NO: 2) (see Figure 4). The WPC was prepared according to the methodology set out in example 1 of WO 99/65326.

**Acute or short term effects** of agents were determined by treating adult SHRs with the agent and then studying their blood pressure responses over the following hours. The study detects if an agent lowers blood pressure that is already elevated.

### Results

Short term dosing of SHRs with 3.6g/kg body weight of WPI hydrolysate (Figure 3) resulted in a significant reduction in blood pressure compared to controls rats (p=0.0378). The reduction in blood pressure was similar to that achieved in rats dosed with 6.0g/kg body weight WPC hydrolysate (Figure 2). Thus, the WPI hydrolysate appears to be more active than the comparative WPC hydrolysate so that approximately 50% less of the WPI hydrolysate is required to give a similar decrease in blood pressure (systolic) in mature SHRs.

Short term dosing of SHRs with 165mg/kg body weight peptide MKG (Figure 4) showed a much greater reduction in blood pressure compared to control rats (p=0.0021 after 4 hours; p<0.0001 after 8 hours) as well as compared to the mild WPI hydrolysate and mild WPC hydrolysate.

### Conclusions

The process of mildly hydrolysing WPI according to the present invention provides useful WPI hydrolysates which show significant functional improvement over the WPC hydrolysates of WO 99/65326. In particular, the present WPI hydrolysates comprise more active peptides with significantly less WPI hydrolysate required to decrease blood pressure in vivo to the same degree as a WPC hydrolysate. WPI can be hydrolysed for longer without losing acceptable flavour characteristics and whilst maintaining the increased ACE-I activity. Thus, overall, the process of the present invention provides a bioactive whey protein hydrolysate which is improved in flavour, ACE-I activity and functionality compared to that of WO 99/65326.

### INDUSTRIAL APPLICATION

The process of the present invention is useful to produce the novel whey protein hydrolysate which has surprising beneficial properties and can be used as a food or medicine as an anti-hypertensive.

It will be appreciated that it is not intended to limit the invention to the aforementioned examples only, many variations, such as might readily occur to a person skilled in the art, being possible, without departing from the scope of the appended claims.

### REFERENCES

Bernal V & Jelen P (1989). Effectiveness of lactose hydrolysis in Cottage cheese whey for the development of whey drinks. Milchwissenchqft 44: 222-225
FR 2309154, 30 December 1976 Fromageries Bel La Vache Qui (From), France.
US 3970520, 20 July 1976, General Electric Co, USA.
EP0117047, 29 August 1984, General Foods Corporation, USA.
Maubois J L, Léonil J, Trouvé R & Bouhallab S (1991) Les peptides du lait A activité physiologique III. Peptides du lait A effect cardiovasculaire: activites antithrombotique et antihypertensive. Lait, 71, 249-255.
JP 4282400, 7 October 1992, Calpis Shokuhin Kogyo KK, Japan.
EP065663, 1 December 1982, Miles Laboratories Incorporated, USA.
JP 8056568, 17 August 1994. Morinaga Milk Co Ltd Japan.
EP4745506, 11 March 1992, Morinaga Milk Co Ltd, Japan.
Mullally M M, Meisel H & FitzGerald R J (1997) Identification of a novel angiotensin-I-converting enzyme inhibitory peptide corresponding to a tryptic fragment of bovine β-lactoglobulin. Federation of European Biochemical Societies Letters, 402, 99-101.
Nakamura Y, Yamamoto N, Sakai K & Takano T (1994) Antihypertensive effect of the peptides derived from casein by an extracellular proteinase from Lactobacillus helveticus CP790. Journal of Dairy Science, 77, 917-922.
Roy G (1992). Bitterness: reduction and inhibition. Trends in Food Science and Technology 3: 85-91
Roy G (1997). Modifying bitterness: Mechanism, ingredients and applications. Technomic Publishers, Lancaster, UK.
US 4358464, 9 September 1982, Superior Dairy Company, USA.
Yamamoto N (1997). Antihypertensive peptides derived from food proteins. Biopolymers 43: 129-134.
Frister H, Meisel H & Schlimme E (1988). OPA method modified by use of N,N-dimethyl-2-mercaptoethylammoniumchloride as thiol component. Freesenius Z Anal Chem. 330, 631-633
Cushman D W & Cheung H S (1971). Spectrophotometric assay and properties of the angiotension converting enzyme in rabbit lung. Biochem Pharmacol 20: 1637-1648.
WO 9965326, 23rd December, 1991, New Zealand Dairy Board, NZ.

## Claims

1. A process for preparing an improved whey protein hydrolysate containing bioactive peptides comprising hydrolysing a whey protein isolate (WPI) with one or more enzymes **characterised in that**:
i) the enzyme is a heat labile protease;
ii) the hydrolysis is conducted at a temperature of between about 30°C and 65°C at a pH of about 3.5 to about 9.0 when said enzyme is a neutral protease, at a pH of about 2.5 to about 6.0 where said enzyme is an acid protease;
iii) the hydrolysis is terminated when a degree of hydrolysis of no greater than about 10% has been reached; and
iv) the hydrolysis is terminated by deactivating said one or more enzymes under conditions to avoid substantial denaturation of peptides or residual proteins in said hydrolysate, wherein said deactivation conditions are selected from the group consisting of; heat deactivation comprising heating said hydrolysate for up to ten seconds to a temperature up to about 100°C;
or when said hydrolysis is conducted at a temperature of below about 65°C, said heat deactivating step is conducted at about 65°C to about 70°C for from about 10 seconds to about 15 minutes;
or when said hydrolysis is conducted at a temperature below about 60°C, said heat deactivation is conducted at about 60°C to about 65°C for from about 10 seconds up to about 30 minutes;
or by altering the pH of said whey-protein containing substrate to a pH at which said protease is not active;
or by subjecting said hydrolysate to ultrafiltration with an ultrafiltration membrane having a nominal molecular weight cutoff in the range of about 10-500 kDa,
wherein the product of the process is highly soluble.

2. A process according to claim 1, wherein the enzyme is selected from the group consisting or Protease P6, Protease A, Protease M, Peptidase, Neutrase, Validase, AFP 2000, and any other heat labile protease.

3. A process as claimed in claim 1, wherein said ultrafiltration membrane has a nominal molecular weight cut off in the range of about 10-200 kDa.

4. A process as claimed in any one of the preceding claims, wherein said enzyme is immobilised on an inert support during said hydrolysis step ii).

5. A process as claimed in claim 4, wherein said inert support is a methacrylate-based functional polymer, carrageenan particles, chitosan particles or any other suitable inert support material.

6. A process as claimed in any one of the preceding claims, wherein the degree of hydrolysis is from about 3% to about 10%.

7. A process as claimed in claim 6, wherein the degree of hydroysis is from about 3% to about 5%.

8. A process as claimed in any one of the preceding claims, wherein the whey protein hydrolysate so produced comprises one or more bioactive peptides selected from the group consisting of SAP (SEQ ID NO: 1), VSLPEW (SEQ ID NO: 5), INYWL (SEQ ID NO: 6), LKPTPEGDLEIL (SEQ ID NO: 7) and LKGYGGVSLPEW (SEQ ID NO: 8).

9. A process as claimed in any one of the preceding claims, wherein the whey protein hydrolysate so prepared comprises at least one bioactive peptide selected from the group consisting of LIVTQ (SEQ ID NO: 4), MKG (SEQ ID NO: 2) and ALPMH (SEQ ID NO: 3), in combination with at least one bioactive peptide selected from the group comprising SAP (SEQ ID NO: 1), VSLPEW (SEQ ID NO: 5), INYWL (SEQ ID NO: 6), LKPTPEGDLEIL (SEQ ID NO: 7) and LKGYGGVSLPEW (SEQ ID NO: 8).

10. A pharmaceutical composition comprising at least one bioactive peptide selected from the group consisting of SAP (SEQ ID NO: 1), VSLPEW (SEQ ID NO: 5), INYWL (SEQ ID NO: 6), LKPTPEGDLEIL (SEQ ID NO: 7) and LKGYGGVSLPEW (SEQ ID NO: 8) in combination with the bioactive peptide MKG (SEQ ID NO: 2) together with a pharmaceutically acceptable carrier.

11. A non-bitter, highly soluble WPI hydrolysate product containing bioactive peptides, prepared by the process of claim 8 or 9.

12. A product as claimed in claim 11 wherein the degree of hydrolysis of the WPI is about 3% to about 5%.

13. A product as claimed in claim 12, wherein the main particle size of whey proteins in the product is less than about 30 microns.

14. A product as claimed in claim 13, wherein the main particle size is less than about 3 microns.

15. A product as claimed in any one of claims 11 to 14 which is clear or white in solution.

16. A product as claimed in any one of claims 11 to 15 wherein one or more of said bioactive peptides is selected from the group consisting of SAP (SEQ ID NO: 1), VSLPEW (SEQ ID NO: 5), INYWL (SEQ ID NO: 6), LKPTPEGDLEIL (SEQ ID NO: 7) and LKGYGGVSLPEW (SEQ ID NO: 8).

17. A product as claimed in any one of claims 11 to 15 comprising at least one bioactive peptide selected from the group consisting of LIVTQ (SEQ ID NO: 4), MKG (SEQ ID NO: 2) and ALPMH (SEQ ID NO: 3), in combination with at least one bioactive peptide selected from the group comprising SAP (SEQ ID NO: 1), VSLPEW (SEQ ID NO: 5), INYWL (SEQ ID NO: 6), LKPTPEGDLEIL (SEQ ID NO: 7) and LKGYGGVSLPEW (SEQ ID NO: 8).

18. A food product containing a WPI hydrolysate product as claimed in any one of claims 11 to 17.

19. A use of a product as claimed in any one of claims 11 to 17 in the manufacture of a medicament for treating or preventing hypertension in a patient in need thereof.

20. A pharmaceutical composition comprising the product of any one of claims 11 to 17 together with a pharmaceutically acceptable carrier.

21. A hydrolysate comprising one or more peptides selected from SAP (SEQ ID NO: 1), VSLPEW (SEQ ID NO: 5), INYWL (SEQ ID NO: 6), LKPTPEGDLEIL (SEQ ID NO: 7) and LKGYGGVSLPEW (SEQ ID NO: 8).

22. A hydrolysate comprising a mixture of at least one first peptide and at least one second peptide, wherein the first peptide is selected from the group consisting of LIVTQ (SEQ ID NO: 4), MKG (SEQ ID NO: 2) and ALPMH (SEQ ID NO: 3), and wherein the second peptide is selected from the group comprising SAP (SEQ ID NO: 1), VSLPEW (SEQ ID NO: 5), INYWL (SEQ ID NO: 6), LKPTPEGDLEIL (SEQ ID NO: 7) and LKGYGGVSLPEW (SEQ ID NO: 8).

23. A pharmaceutical composition comprising the hydrolysate as claimed in claim 21 or 22 together with a pharmaceutically acceptable carrier.

24. A use of a hydrolysate as claimed in claim 21 or 22 for the manufacture of a medicament for treating or preventing hypertension in a patient in need of such treatment.

25. A process according to claim 8 or claim 9 further comprising a step of combining a pharmaceutically acceptable carrier to produce a pharmaceutical composition.

## Patentansprüche

1. Verfahren zur Zubereitung eines verbesserten Molkeproteinhydrolysats, das bioaktive Peptide enthält, umfassend das Hydrolysieren eines Molkeproteinisolats (WPI) mit einem oder mehreren Enzymen, **dadurch gekennzeichnet, dass**
i) das Enzym eine wärmelabile Protease ist;
ii) die Hydrolyse bei einer Temperatur zwischen ungefähr 30°C und 65°C bei einem pH von ungefähr 2,5 bis ungefähr 6,0 ausgeführt wird, wobei das Enzym eine Säureprotease ist;
iii) die Hydrolyse beendet wird, wenn ein Hydrolysegrad von höchstens ungefähr 10% erreicht worden ist; und
iv) die Hydrolyse durch Deaktivieren des einen oder der mehreren Enzyme unter Bedingungen zur Vermeidung einer wesentlichen Denaturierung von Peptiden oder Restproteinen in dem Hydrolysat beendet wird, wobei die Deaktivierungsbedingungen aus der Gruppe ausgewählt werden, bestehend aus Wärmedeaktivierung, die das Erhitzen des Hydrolysats bis zu zehn Sekunden lang auf eine Temperatur von bis zu ungefähr 100°C umfasst;
oder, wenn die Hydrolyse bei einer Temperatur von unterhalb ungefähr 65°C ausgeführt wird, der Wärmedeaktivierungsschritt ungefähr 10 Sekunden bis ungefähr 15 Minuten lang bei ungefähr 65°C bis ungefähr 70°C ausgeführt wird;
oder, wenn die Hydrolyse bei einer Temperatur von unterhalb ungefähr 60°C ausgeführt wird, die Wärmedeaktivierung ungefähr 10 Sekunden bis ungefähr 30 Minuten lang bei ungefähr 60°C bis ungefähr 65°C ausgeführt wird;
oder durch Ändern des pH des Molkeprotein enthaltenden Substrats auf einen pH, bei dem die Protease nicht aktiv ist;
oder indem das Hydrolysat einer Ultrafiltration mit einer Ultrafiltrationsmembran unterzogen wird, die eine nominale Molekulargewichtsgrenze im Bereich von ungefähr 10-500 kDa aufweist,
wobei das Produkt des Verfahrens stark löslich ist.

2. Verfahren nach Anspruch 1, wobei das Enzym ausgewählt ist aus der Gruppe, bestehend aus Protease P6, Protease A, Protease M, Peptidase, Neutrase, Validase, AFP 2000 und einer beliebigen anderen wärmelabilen Protease.

3. Verfahren nach Anspruch1, wobei die Ultrafiltrationsmembran eine nominale Molekulargewichtsgrenze im Bereich von ungefähr 10-200 kDa aufweist.

4. Verfahren nach irgendeinem der vorhergehenden Ansprüche, wobei das Enzym während des Hydrolyseschritts ii) auf einem inerten Trägerstoff immobilisiert ist.

5. Verfahren nach Anspruch4, wobei der inerte Trägerstoff ein funktionelles Polymer auf Methacrylatbasis, Carrageenpartikel, Chitosanpartikel oder irgendein anderes geeignetes Inertträgerstoffmaterial ist.

6. Verfahren nach irgendeinem der vorhergehenden Ansprüche, wobei der Grad der Hydrolyse ungefähr 3% bis ungefähr 10% beträgt.

7. Verfahren nach Anspruch 6, wobei der Grad der Hydrolyse ungefähr 3% bis ungefähr 5% beträgt.

8. Verfahren nach irgendeinem der vorhergehenden Ansprüche, wobei das derart hergestellte Molkeproteinhydrolysat eines oder mehrere bioaktive Peptide umfasst, die ausgewählt sind aus der Gruppe, bestehend aus SAP (SEQ ID NO: 1), VSLPEW (SEQ ID NO: 5), INYWL (SEQ ID NO: 6), LKPTPEGDLEIL (SEQ ID NO: 7) und LKGYGGVSLPEW (SEQ ID NO: 8).

9. Verfahren nach irgendeinem der vorhergehenden Ansprüche, wobei das derart hergestellte Molkeproteinhydrolysat zumindest ein bioaktives Peptid umfasst, das ausgewählt ist aus der Gruppe, bestehend aus LIVTQ (SEQ ID NO: 4), MKG (SEQ ID NO: 2) und ALPMH (SEQ ID NO: 3) in Kombination mit zumindest einem bioaktiven Peptid, das ausgewählt ist aus der Gruppe, umfassend SAP (SEQ ID NO: 1), VSLPEW (SEQ ID NO: 5), INYWL (SEQ ID NO: 6), LKPTPEGDLEIL (SEQ ID NO: 7) und LKGYGGVSLPEW (SEQ ID NO: 8).

10. Pharmazeutische Zusammensetzung, umfassend zumindest ein bioaktives Peptid, das ausgewählt ist aus der Gruppe, bestehend aus SAP (SEQ ID NO: 1), VSLPEW (SEQ ID NO: 5), INYWL (SEQ ID NO: 6), LKPTPEGDLEIL (SEQ ID NO: 7) und LKGYGGVSLPEW (SEQ ID NO: 8) in Kombination mit dem bioaktiven Peptid MKG (SEQ ID NO: 2) zusammen mit einem pharmazeutisch annehmbaren Träger.

11. Nicht-bitteres, stark lösliches WPI-Hydrolysatprodukt, das bioaktive Peptide enthält, das durch das Verfahren nach Anspruch 8 oder 9 zubereitet ist.

12. Produkt nach Anspruch 11, wobei der Grad der Hydrolyse des WPI ungefähr 3% bis ungefähr 5% beträgt.

13. Produkt nach Anspruch 12, wobei die Hauptpartikelgröße von Molkeproteinen in dem Produkt weniger als ungefähr 30 µm beträgt.

14. Produkt nach Anspruch 13, wobei die Hauptpartikelgröße weniger als ungefähr 3 µm beträgt.

15. Produkt nach irgendeinem der Ansprüche 11 bis 14, das in Lösung klar oder weiß ist.

16. Produkt nach irgendeinem der Ansprüche 11 bis 15, wobei eines oder mehrere der bioaktiven Peptide ausgewählt ist aus der Gruppe, bestehend aus SAP (SEQ ID NO: 1), VSLPEW (SEQ ID NO: 5), INYWL (SEQ ID NO: 6), LKPTPEGDLEIL (SEQ ID NO: 7) und LKGYGGVSLPEW (SEQ ID NO: 8).

17. Produkt nach irgendeinem der Ansprüche 11 bis 15, umfassend zumindest ein bioaktives Peptid, das ausgewählt ist aus der Gruppe, bestehend aus LIVTQ (SEQ ID NO: 4), MKG (SEQ ID NO: 2) und ALPMH (SEQ ID NO: 3) in Kombination mit zumindest einem bioaktiven Peptid, das ausgewählt ist aus der Gruppe, umfassend SAP (SEQ ID NO: 1), VSLPEW (SEQ ID NO: 5), INYWL (SEQ ID NO: 6), LKPTPEGDLEIL (SEQ ID NO: 7) und LKGYGGVSLPEW (SEQ ID NO: 8).

18. Lebensmittelprodukt, enthaltend ein WPI-Hydrolysatprodukt nach irgendeinem der Ansprüche 11 bis 17.

19. Verwendung eines Produkts nach irgendeinem der Ansprüche 11 bis 17 bei der Herstellung eines Medikaments zur Behandlung oder Verhinderung von Hypertonie bei einem diese benötigenden Patienten.

20. Pharmazeutische Zusammensetzung, umfassend das Produkt nach irgendeinem der Ansprüche 11 bis 17 zusammen mit einem pharmazeutisch annehmbaren Träger.

21. Hydrolysat, umfassend eines oder mehrere Peptide, die ausgewählt sind aus SAP (SEQ ID NO: 1), VSLPEW (SEQ ID NO: 5), INYWL (SEQ ID NO: 6), LKPTPEGDLEIL (SEQ ID NO: 7) und LKGYGGVSLPEW (SEQ ID NO: 8).

22. Hydrolysat, umfassend ein Gemisch aus zumindest einem ersten Peptid und zumindest einem zweiten Peptid, wobei das erste Peptid ausgewählt ist aus der Gruppe, bestehend aus LIVTQ (SEQ ID NO: 4), MKG (SEQ ID NO: 2) und ALPMH (SEQ ID NO: 3), und wobei das zweite Peptid ausgewählt ist aus der Gruppe, umfassend SAP (SEQ ID NO: 1), VSLPEW (SEQ ID NO: 5), INYWL (SEQ ID NO: 6), LKPTPEGDLEIL (SEQ ID NO: 7) und LKGYGGVSLPEW (SEQ ID NO: 8).

23. Pharmazeutische Zusammensetzung, umfassend das Hydrolysat nach Anspruch 21 oder 22 zusammen mit einem pharmazeutisch annehmbaren Träger.

24. Verwendung eines Hydrolysats nach Anspruch 21 oder 22 für die Herstellung eines Medikaments zur Behandlung oder Verhinderung von Hypertonie bei einem Patienten, der einer solchen Behandlung bedarf.

25. Verfahren nach Anspruch 8 oder Anspruch 9, weiterhin umfassend einen Schritt des Kombinierens eines pharmazeutisch annehmbaren Trägers zur Herstellung einer pharmazeutischen Zusammensetzung.

## Revendications

1. Un processus de préparation d'un hydrolysat amélioré de protéines du lactosérum contenant des peptides bioactifs comprenant l'hydrolyse d'un isolat pur de protéines du lactosérum (WPI) avec une ou plusieurs enzymes **caractérisées en ce que** :
i) l'enzyme est une protéase labile à la chaleur ;
ii) l'hydrolyse est réalisée à un température comprise entre environ 30°C et 65°C à un pH de 3,5 environ à 9,0 environ lorsque ladite enzyme est une protéase neutre, à un pH de 2,5 environ à 6,0 environ lorsque ladite enzyme est une protéase acide ;
iii) l'hydrolyse est terminée lorsque un taux d'hydrolyse qui n'est pas supérieur à environ 10% est atteint ; et
iv) l'hydrolyse se termine par la désactivation de ladite ou desdites enzymes dans des conditions qui empêchent la dénaturation substantielle des peptides ou des protéines résiduelles dudit hydrolysat, dans lequel lesdites conditions de désactivation sont choisies dans le groupe formé par : la désactivation par la chaleur comprenant le chauffage dudit hydrolysat jusqu'à dix secondes à une température allant jusqu'à 100°C environ ;
ou lorsque ladite hydrolyse est réalisée à une température inférieure à environ 65°C, ladite étape de désactivation par la chaleur est réalisée de 65°C environ à 70°C environ pendant 10 secondes environ à 15 minutes environ ;
ou lorsque ladite hydrolyse est réalisée à une température inférieure à environ 60°C, ladite étape de désactivation par la chaleur est réalisée de 60°C environ à 65°C environ pendant 10 secondes environ jusqu'à environ 30 minutes ;
ou par l'altération du pH du substrat contenant lesdites protéines de lactosérum à un pH auquel ladite protéase n'est pas active ;
ou en soumettant ledit hydrolysat à l'ultrafiltration avec une membrane d'ultrafiltration ayant un seuil de coupure de poids moléculaire nominal de l'ordre de 10-500 kDa environ, dans lequel le produit du processus est hautement soluble.

2. Un processus conforme à la revendication 1, dans lequel l'enzyme est choisi dans le groupe formé par la Protéase P6, la Protéase A, la Protéase M, la Peptidase, la Neutrase, la Validase, l'AFP 2000, et tout autre protéase labile à la chaleur.

3. Un processus conforme à la revendication 1, dans lequel ladite membrane d'ultrafiltration membrane a un seuil de coupure de poids moléculaire nominal de l'ordre de 10-200 kDa environ.

4. Un processus conforme à l'une quelconque des revendications précédentes, dans lequel ladite enzyme est immobilisée sur un support inerte au cours de l'étape ii) de ladite hydrolyse.

5. Un processus conforme à la revendication 4, dans lequel ledit support inerte est un polymère fonctionnel à base de méthacrylate, des particules de carraghénane, des particules de chitosane ou tout autre matériau de support inerte acceptable.

6. Un processus conforme à l'une quelconque des revendications précédentes, dans lequel le taux d'hydrolyse est compris entre environ 3% et environ 10%.

7. Un processus conforme à la revendication 6, dans lequel le taux d'hydrolyse est compris entre environ 3% et environ 5%.

8. Un processus conforme à l'une quelconque des revendications précédentes, dans lequel l'hydrolysat de protéines du lactosérum ainsi produit comprend un ou plusieurs peptides bioactifs choisi(s) dans le groupe formé par SAP (SEQ ID NO: 1), VSLPEW (SEQ ID NO: 5), INYWL (SEQ ID NO: 6), LKPTPEGDLEIL (SEQ ID NO: 7) et LKGYGGVSLPEW (SEQ ID NO: 8).

9. Un processus conforme à l'une quelconque des revendications précédentes, dans lequel l'hydrolysat de protéines du lactosérum ainsi préparé comprend au moins un peptide bioactif choisi dans le groupe formé par LIVTQ (SEQ ID NO: 4), MKG (SEQ ID NO: 2) et ALPMH (SEQ ID NO: 3), en combinaison avec au moins un peptide bioactif choisi dans le groupe comprenant SAP (SEQ ID NO: 1), VSLPEW (SEQ ID NO: 5), INYWL (SEQ ID NO: 6), LKPTPEGDLEIL (SEQ ID NO: 7) et LKGYGGVSLPEW (SEQ ID NO: 8).

10. Une composition pharmaceutique comprenant au moins un peptide bioactif choisi dans le groupe formé par SAP (SEQ ID NO: 1), VSLPEW (SEQ ID NO: 5), INYWL (SEQ ID NO: 6), LKPTPEGDLEIL (SEQ ID NO: 7) et LKGYGGVSLPEW (SEQ ID NO: 8) en combinaison avec le peptide bioactif MKG (SEQ ID NO: 2) associés à un transport pharmaceutiquement approprié.

11. Un hydrolysat de WPI non amer, hautement soluble contenant des peptides bioactifs, préparé selon le processus de la revendication 8 ou 9.

12. Un produit conforme à la revendication 11 dans lequel le taux d'hydrolyse du WPI est de 3% environ à 5% environ.

13. Un produit conforme à la revendication 12, dans lequel la taille principale des particules des protéines du lactosérum dans le produit est inférieure à 30 microns environ.

14. Un produit conforme à la revendication 13, dans lequel la taille principale des particules est inférieure à 3 microns environ.

15. Un produit conforme à l'une quelconque des revendications 11 à 14 qui est clair ou blanc en solution.

16. Un produit conforme à l'une quelconque des revendications 11 à 15 dans lequel un ou plusieurs desdits peptides bioactifs est choisi dans le groupe formé par SAP (SEQ ID NO: 1), VSLPEW (SEQ ID NO: 5), INYWL (SEQ ID NO: 6), LKPTPEGDLEIL (SEQ ID NO: 7) et LKGYGGVSLPEW (SEQ ID NO: 8).

17. Un produit conforme à l'une quelconque des revendications 11 à 15 comprenant au moins un peptide bioactif choisi dans le groupe formé par LIVTQ (SEQ ID NO: 4), MKG (SEQ ID NO: 2) et ALPMH (SEQ ID NO: 3), en combinaison avec au moins un peptide bioactif choisi dans le groupe comprenant SAP (SEQ ID NO: 1), VSLPEW (SEQ ID NO: 5), INYWL (SEQ ID NO: 6), LKPTPEGDLEIL (SEQ ID NO: 7) et LKGYGGVSLPEW (SEQ ID NO: 8).

18. Un produit alimentaire contenant un produit hydrolysat de WPI conforme à l'une quelconque des revendications 11 à 17.

19. L'utilisation d'un produit conforme à l'une quelconque des revendications 11 à 17 dans la fabrication d'un médicament pour le traitement ou la prévention de l'hypertension chez un patient qui en a besoin.

20. Une composition pharmaceutique comprenant le produit de l'une quelconque des revendications 11 à 17 associé à un transport pharmaceutiquement acceptable.

21. Un hydrolysat comprenant un ou plusieurs peptides choisis parmi SAP (SEQ ID NO: 1), VSLPEW (SEQ ID NO: 5), INYWL (SEQ ID NO: 6), LKPTPEGDLEIL (SEQ ID NO: 7) et LKGYGGVSLPEW (SEQ ID NO: 8).

22. Un hydrolysat comprenant un mélange d'au moins un premier peptide et d'au moins un second peptide, dans lequel le premier peptide est choisi dans le groupe formé par LIVTQ (SEQ ID NO: 4), MKG (SEQ ID NO: 2) et ALPMH (SEQ ID NO: 3), et dans lequel le second peptide est choisi dans le groupe comprenant SAP (SEQ ID NO: 1), VSLPEW (SEQ ID NO: 5), INYWL (SEQ ID NO: 6), LKPTPEGDLEIL (SEQ ID NO: 7) et LKGYGGVSLPEW (SEQ ID NO: 8).

23. Une composition pharmaceutique comprenant l'hydrolysat conforme à la revendication 21 ou 22 associé à un transport pharmaceutiquement acceptable.

24. L'utilisation d'un hydrolysat conforme à la revendication 21 ou 22 pour la fabrication d'un médicament pour le traitement ou la prévention de l'hypertension chez un patient qui a besoin d'un tel traitement.

25. Un processus conforme à la revendication 8 ou à la revendication 9 comprenant aussi un étape de combinaison avec un transport pharmaceutiquement acceptable pour produire une composition pharmaceutique.


### SEQUENCE LISTING

<110> NEW ZEALAND DAIRY BOARD
<120> Improved Bioactive Whey Protein Hydrolysate
<130> P425133 CJE
<140>
   <141>
<150> NZ 506866
   <151> 2000-09-11
<160> 8
<170> PatentIn Ver. 2.1
<210> 1
   <211> 3
   <212> PRT
   <213> Peptide
<400> 1
   Ser Ala Pro
     1
<210> 2
   <211> 3
   <212> PRT
   <213> Peptide
<400> 2
   Met Lys Gly
   1
<210> 3
   <211> 5
   <212> PRT
   <213> Peptide
<400> 3
   Ala Leu Pro Met His
   1 5
<210> 4
   <211> 5
   <212> PRT
   <213> Peptide
<400> 4
   Leu Ile Val Thr Gln
   1 5
<210> 5
   <211> 6
   <212> PRT
   <213> Peptide
<400> 5
   Val Ser Leu Pro Glu Trp
   1 5
<210> 6
   <211> 5
   <212> PRT
   <213> Peptide
<400> 6
   Ile Asn Tyr Trp Leu
   1 5
<210> 7
   <211> 12
   <212> PRT
   <213> Peptide
<400> 7
   Leu Lys Pro Thr Pro Glu Gly Asp Leu Glu Ile Leu
    1               5                   10
<210> 8
   <211> 12
   <212> PRT
   <213> Peptide
<400> 8
   Leu Lys Gly Tyr Gly Gly Val Ser Leu Pro Glu Trp
    1               5                   10